# EUROPEAN PATENT SPECIFICATION

(11) **EP 1 127 121 B1**
(45) Date of publication and mention of the grant of the patent: **14.12.2005**
(21) Application number: 99956693.8
(22) Date of filing: 27.10.1999
(51) Int. Cl.: C12N 15/12, C12N 15/31, C12N 15/53, C12N 15/867, C12N 9/02, C07K 14/36, C07K 14/47, A61K 48/00, A61P 17/00, A61P 17/14

(54) **CONSTRUCTION D'UNE RETROVIRUS EXPRIMANT LE LOCUS MEL DE STREPTOMYCES, ET SON EXPRESSION DANS DES CELLULES MAMMIFERES**
HERSTELLUNG EINES RETROVIRUS, WELCHES DEN MEL-LOKUS VON STREPTOMYCES ENTHÄLT, SOWIE DESSEN EXPRESSION IN SÄUGETIERZELLEN
CONSTRUCTION D'UNE RETROVIRUS EXPRIMANT LE LOCUS MEL DE STREPTOMYCES, ET SON EXPRESSION DANS DES CELLULES MAMMIFERES

(30) Priority: 27.10.1998 US 105725 P; 27.10.1998 US 105831 P
(43) Date of publication of application: 29.08.2001
(73) Proprietor: ANTICANCER, INC., San Diego, CA 92111 (US)
(72) Inventor: ZHAO, Ming, San Diego, CA 92122 (US)
(74) Representative: Roques, Sarah Elizabeth
(86) International application number: PCT/US1999/025118
(87) International publication number: WO 2000/024895

(56) References cited:
- WO-A-94/22468
- WO-A-95/13386
- WO-A-96/12506
- WO-A-97/27297
- WO-A-98/46208
- US-A- 5 753 263
- LI L ET AL.: "The feasibility of targeted selective gene therapy of the hair follicle" NATURE MEDICINE, vol. 1, no. 7, July 1995 (1995-07), pages 705-706, XP002140346 ISSN: 1078-8956
- MACLACHLAN T K ET AL: "Cyclins, cyclin-dependent kinases and Cdk inhibitors: implications in cell cycle control and cancer" CRITICAL REVIEWS IN EUKARYOTIC GENE EXPRESSION, vol. 5, no. 2, 1995, pages 127-156, XP000572011 ISSN: 0029-0726
- HOFFMAN R M: "The hair follicle as a gene therapy target" NATURE BIOTECHNOLOGY, vol. 18, January 2000 (2000-01), pages 20-21, XP002140347 ISSN: 1087-0156
- DIRKS ET AL: 'Dicistronic transcription units for gene expression in mammalian cells' GENE vol. 128, no. 2, 30 June 1993, pages 247 - 249

## Description

### Technical Field

The invention generally relates to compositions and methods for treating disorders related to tyrosinase gene expression and melanin biosynthesis. In addition, the invention relates to a model for pigmentation of hair.

### Background Art

Treatment of the hair and skin with various creams or lotions with biologically active ingredients to improve hair growth and pigmentation has generally been unsatisfactory. A wide variety of externally applied agents available are said to improve body, flexibility, curl and hair color. These have limited and only short term usefulness. In particular, coloring hair with various dyes requires frequent repetitions and is not always natural in appearance. The invention provides improved alternatives, focused on tyrosinase and cell cycle inhibitors.

Tyrosinase is a ubiquitously distributed copper-containing monoxygenase that is essential for melanin biosynthesis in pigment cells. It catalyzes the conversion of tyrosine to dihydroxyphenylalanine (DOPA) and the conversion of DOPA to dopaquinone, referred to as tyrosine hydroxylase activity and DOPA oxidase activity, respectively.

Disorders of tyrosinase gene expression and melanin biosynthesis are related to many diseases involving pigmentation such as albinism, hair pigment loss, and vitellego. Tyrosinase is a key enzyme for melanin synthesis in vertebrate pigment cells, melanocytes, and retinal pigment epithelial cells. Tyrosinase is absent in human white hair bulbs, as well as in albino epithelial cells. Thus, the loss of tyrosinase could be the basis of pigment loss in hair. It is also believed that tyrosinase activity is abnormal in Parkinson's disease. Tyrosinase is expressed in brain cells in the substantia nigra, forebrain, and midbrain, thus tyrosinase could be implicated in neuromelanin formation in the substantia nigra and thus related to neurodegenerative disorders.

The murine and human tyrosinase genes have been cloned. (Kwon *et al. Proc Natl Acad Sci USA* (1987) 84:7473-7477; Yamamoto *et al. Jpn J Genet* (1989) 64:121-135; Ruppert *et al. EMBO J* (1988) 7:2715-2722. The structural genes for human and murine tyrosinase have long DNA sequences. In mice, the structural gene for tyrosinase is located on the c-locus of chromosome 7, which spans a region of more than 70 kb. In humans, the tyrosinase gene is located on the c-locus on chromosome 11. It has been reported that only the full complement of five exons were able to confer tyrosinase enzyme activity to tyrosinase-negative cells.

Murine and human tyrosinase cDNAs have been expressed in mammalian cells after transfection. See Muller *et al*. (1988), Tomita *et al*. (1989), Yamamoto *et al*. (1989) *supra*, Geibal *et al*. (1990), Porter *et al*. *Gene* (1991) 97:277-282; Mishima *et al*. (1993), and U.S. Patent No. 5,753,263. Several cDNA clones of the mouse tyrosinase have been isolated independently. Among those cDNA clones, there are several differences in the deduced amino acid sequence. Muller *et al*. (1988) *supra* reported that one of their cDNA clones, pmcTYr1, possessed the coding capacity for tyrosinase, and they demonstrated that pmcTYr1 could be inserted in cultured human amelanotic melanoma cells. However, they did not report any pigment production. On the other hand, Yamamoto *et al*. (1989) *supra* constructed the mouse tyrosinase cDNA minigene, mg-Tyr1-J which was ligated with a genomic 5'-non-coding flanking sequence and transfected cultured albino melanocytes, which produced tyrosinase and melanin.

The tyrosinase gene in albino mice has a single base-pair mutation in exon 1 which results in a nonfunctional tyrosinase protein and absence of melanin in the albino melanocytes. Melanin has been produced in amelanotic melanocytes in transgenic albino mice whose fertilized eggs were inserted with a normal mouse tyrosinase transgene, and melanin was seen in the hair bulbs, hair shafts, choroid, and pigment epithelium. Tanaka *et al. Development* (1990) 108:223-227; Beerman *et al. Pigment Cell Research* (1992) 5:295-299. Human tyrosinase cDNA has been used to transfect amelanotic melanoma cells and induce melanin production. Ando *et al. J Invest Dermatol* (1993) 101:864-870.

The human and murine tyrosinase constructs are difficult to manipulate due to their complexity and size. Many species of *Streptomyces* are also capable of forming melanin due to the expression of tyrosinase from the *mel* operon. See Kuster *et al.* (1976) Chromogenicity of actinomycetes. *ACTINOMYCETES:* THE BOUNDARY OF MICROORGANISMS. Ed. T. Arai, Tokyo: Toppan Co. 43-45. The 1.2 kb *mel* locus of *S. antibioticus* has been cloned and sequenced and shown to contain a structural tyrosinase gene and an open reading frame ORF-438. The ORF-438 protein regulates copper incorporation, which is essential for the expression and function of tyrosinase and melanin production in *Streptomyces.* The ORF-438 and tyrosinase genes are transcribed from the same promoter located in the 5'-region adjacent to ORF-438, thereby indicating that these genes form an operon. Bernan *et al. Gene* (1985) 37:101-110. The ORF-438 protein functions as a transactivator of tyrosinase. See Lee *et al. Gene* (1988) 65:71-81. The ORF-438 protein also delivers copper to apotyrosinase to generate active tyrosinase. Production of melanin in *E. coli* from the *mel* locus of *S*. *antibioticus* was dependent on the coexpression of a full length ORF-438 gene as well as the tyrosinase gene. See Della-Cioppa *et al*. *Biotechnology* (1990) 8:634-638.

Advantage has been taken of the characteristics of the *mel* operon which is smaller in size, lacks introns, and exhibits a potential for high expression to construct vectors useful to construct models for pigmentation disorders and for treatment of tyrosinase deficiency.

Use of cell cycle inhibitors to counteract the effects of alopecia by delivering genes encoding such inhibitors to hair follicles in liposomal formulations has been suggested in U.S. Patent No. 5,753,263 issued 19 May 1998. The present invention describes the successful expression of the p21 gene in hair follicles in skin histoculture as monitored by GFP fluorescence. Administration of this cell cycle inhibitor or other cell cycle inhibitors to control alopecia can be performed coincident with or independently of correction of tyrosinase efficiency.

### Summary of the Invention

Generally, the present invention is directed to compositions and methods for treating disorders related to tyrosinase gene expression and melanin biosynthesis, and to model systems for such disorders. It also relates to use of cell cycle inhibitors to control alopecia by effecting expression of these inhibitors in hair follicles.

In one aspect, the invention is directed to a nucleic acid molecule comprising a bicistronic nucleotide sequence for efficient production of a functional *Streptomyces* tyrosinase in mammalian cells comprising a tyrosinase encoding nucleotide sequence and nucleotide sequence encoding ORF-438, coupled through an internal ribosome entry sequence (IRES).

In another aspect, the invention is directed to the expression system of the invention for use in a method for treating a tyrosinase deficiency or pigmentation disorder in a subject which method comprises modifying the cells of the subject with the expression system of the invention. In a preferred embodiment, the cells to be treated are hair follicle cells and the subjects are mammals, such as primates, including humans, domestic animals, and rodents. The expression system can be utilized as the sole method of treatment, but the system may be used in combination with administration of other beneficial compounds such as proteins, pigments, dyes, growth regulators and other compounds which affect the characteristics of skin and hair. Thus, the expression system may be utilized in addition to cell cycle regulating proteins or the multi-drug resistance proteins which confer resistance to chemotherapy-induced alopecia.

In another aspect, the invention is directed to cells modified to contain the expression system of the invention for the expression of active tyrosinase and production of melanin. In another aspect, the invention is directed to a nonhuman animal comprising the modified cells. The invention is also directed to vectors which comprise the nucleotide sequence encoding tyrosinase and a protein for the incorporation of copper.

In another embodiment, the invention is directed to a method to monitor the effect of compounds or protocols on pigment disorders using histocultures of cells containing the expression systems of the invention.

### Brief Description of the Drawings

Figure 1 shows schematically shows how the retroviral vector pL*mel*SN was constructed.
Figure 2 shows the nucleotide sequence of the bicistronic pL*mel*SN insert; as indicated, both the ORF-438 and tyrosinase sequences are preceded by the Kozak sequence and terminated with TAA.
Figure 3 shows tyrosinase activity in pL*mel*SN-transduced packaging cells.

### Detailed Description of the Invention

Tyrosinase is the key enzyme for melanin biosynthesis. Disorders of tyrosinase activity include Parkinson's disease, vitellego and albinism. As described herein, this invention provides a method of genetically modifying tyrosinase expression and melanin production.

Thus, the invention provides uniquely effective protocols and materials for the treatment of disorders related to tyrosinase activity, as well as assay systems for monitoring the production of tyrosinase or melanin in cells. In particular, the invention provides protocols and materials for treating pigmentation disorders, as well as assay systems for monitoring the effect of various treatments on production of tyrosinase or melanin in hair follicle cells.

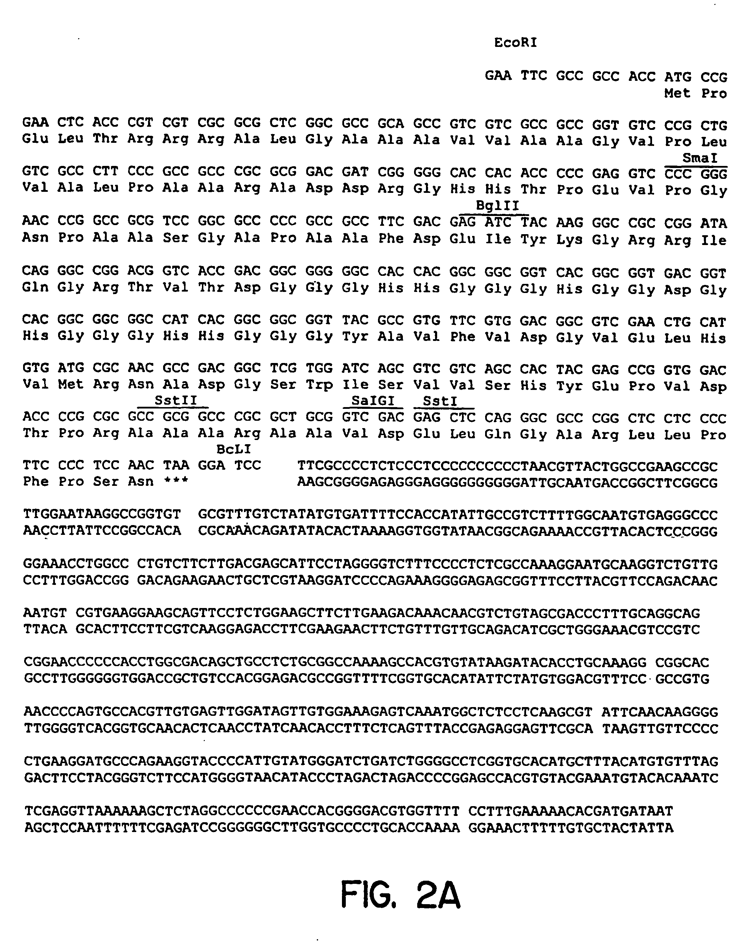

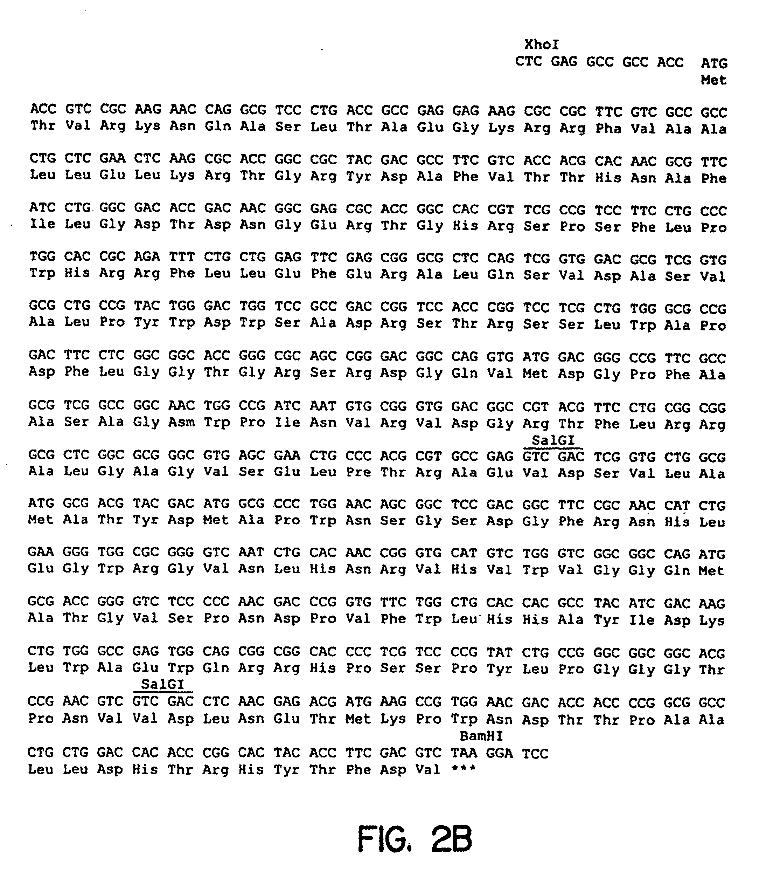

The expression systems employed in the present invention generally comprise a bicistronic nucleotide sequence comprising a modified *Streptomyces mel* locus. The bicistronic nucleotide sequence which comprises a tyrosinase encoding portion, an ORF438 encoding portion and an IRES coupling the two components, can be arranged in either order. That is, as shown in Figure 2, the construct may contain the sequence encoding ORF438 followed by the IRES followed by the gene encoding tyrosinase or, conversely, the tyrosinase encoding portion may be followed by IRES followed by ORF438. Typically, the bicistronic nucleotide sequence is inserted into a vector which contains control sequences operably linked to the bicistronic sequence for delivery to the intended cell, typically hair follicles. However, as is understood in the art, the bicistronic sequence of the invention may be inserted into a host cell so as to effect expression under control of endogenous promoters and terminating sequences. Vectors for delivery of the nucleotide sequence may be designed to replicate extrachromosomally but preferably may effect the insertion of the nucleotide sequence into the genome of the host, and thus may employ endogenous control sequences to effect expression. The nucleotide sequence to be inserted may be coupled to sequences which effect homologous recombination at known targets in the genome; random integration into the genome may be relied upon, or a retroviral vector may be employed. Use of viral vectors especially retroviral vectors containing the expression system of the invention is a preferred method.

Retroviral vectors have been widely used in gene therapy studies. See Jolly *et al.* (1997) Viral Vector Systems for Gene Therapy. THE INTERNET BOOK OF GENE THERAPY. Eds. Sobol, R.E. and Scanlon, K.J. Stamford, CT, Appleton and Lange, pp. 3-16. Retroviral vectors integrate a DNA copy of their genome into the host chromosome, thereby allowing for stable transmission of vector sequences to descendent cells, making long-term expression of transgenes possible. While retroviral vectors are preferred, other viral based methods may also be employed, such as *Herpes simplex* vectors, Wolfe, J.H., *et al. Nature Genetics* (1992) 1:379-384, or adenovirus vectors such as Ad2 or Ad5. These viruses are not oncogenic and are relatively stable and easy to manipulate. The complete genomes of Ad2 and Ad5 are known and there are a number of known mutants that have been made available for genetic therapy in general. Berkner, K.L., *Biotechniques* (1988) 6:612-629. However, while viral-based vectors are preferred, any suitable vector for transformation can be employed.

If viral vectors are used, the specificity of infectivity can be enhanced by including receptor-binding moieties in the vector. It is known in the art to modify viral surface molecules with binding domains of ligands, such as erythropoietin which targets erythroleukemia, heregulin which targets breast cancer and neurotensin which targets the colon. Thus, viral surface molecules may be modified to contain binding domains of ligands which target hair follicle cells or other cells related to hair and skin pigmentation.

The expression system of the invention employs suitable promoters and enhancers. General constitutive promoters such as SV40 or CMV promoters can be included, along with their enhancer elements, or tissue-specific promoters may be used to enhance specificity. Means to construct suitable vectors for delivery of a gene along with provision for its expression are well known in the art.

In order to effect the modification of cells for the expression of tyrosinase and the production of melanin, the expression system or integrating encoding nucleotide sequence must be formulated so as to enter the cell. Integration of the desired nucleotide sequences into viral vectors provides this means of entry. However, other mediators of cellular uptake, such as lipids, or various liposomal type compositions or emulsions may also be employed.

The composition, comprising a nucleotide sequence encoding a protein with tyrosinase activity, preferably operably linked control sequences, or sequences effecting genomic integration, thus may contain a means for effecting cellular uptake. This composition is supplied to the cells, either *in vitro* or *in vivo. In vitro* administration is particularly helpful in assessing, in advance, the efficacy of the therapeutic approach for a particular individual, as well as expression and production levels of the tyrosinase and melanin in the cells.

Additionally, the expression system may be delivered into the skin without a carrier. For example, naked DNA may be introduced into the skin by direct injection, by gene gun, or by using an electric pulse. See Furth *et al. Hybridoma* (1996) 14:149-152; Hengge *et al. Nature Genet* (1995) 110:161-166; Ciernik *et al. Hum Gene Ther* (1996) 7:893-899; Williams *et al. Proc Natl Acad Sci USA* (1991) 88:2726-2730; Zhang *et al. Biochem Biophys Res Commun* (1996) 220:633-636. Topical application of the expression system as naked plasmid DNA comprising a tyrosinase gene and a gene which encodes a protein that regulates copper incorporation such as ORF-438 from *S. antibioticus* is also contemplated as a gene therapy method for treating disorders related to tyrosinase expression and melanin production. The expression system may be applied directly to the skin by the various methods known in the art. See Fan *et al. Nature Biotech* (1990) 17:870-872; Yu *et al. J Invest Dermatology* (1999) 112:370-375; Tang *et al. Nature* (1997) 338:729-730. The expression system may also be delivered by genetic vaccination or polynucleotide vaccination as described by Shi *et al. Vaccine* (1999) 17(17):2136-41, Falo, L. D. *Proc Assoc Am Physicians* (1999) 111(3):211-9, and Tuting *et al. J Invest Dermatol* (1998) 111(2):183-8.

It may be advantageous in some contexts to employ the protein having tyrosinase activity as a fusion protein to a reporter amino acid sequence, most preferably an amino acid sequence which confers fluorescence on the fusion protein. The use of green fluorescent protein (GFP) to confer fluorescence on a fusion protein is well understood in the art; see, for example, Chalfie, M., *et al. Science* (1994) 263:802-805. The expression system may be targeted to the cells of interest, such as hair follicle cells by utilizing liposome-mediated delivery as described in U.S. Pat. No. 5,641,508.

The invention contemplates using the bicistronic nucleotide sequence to treat diseases associated with tyrosinase expression such as Parkinson's disease, albinism, hair pigment loss and vitellego. Similarly, the invention contemplates using the expression system in *in vitro* and animal models to determine the effects of various substances on pigmentation. In both cases, cells which exhibit tyrosinase abnormalities, whether contained *in vitro* or *in vivo,* must be accessed by the bicistronic nucleotide sequence in such a fashion as to exact its expression. For use in evaluating protocols or compounds for their effect on pigmentation, generally the cells are modified *in vitro* or *in vivo* and maintained in a histoculture. Cell cultures can be modified *in vitro* with a variety of methods including electroporation, lipofection, viral infection, osmotic alteration, and the like. They can then be cultured in a three-dimensional matrix as is understood in the art. Alternatively, for either treatment or formation of histocultures, cells can be modified *in vivo* by administering the subject harboring such cells a suitable composition containing the required sequences. Such administration may include the use of "naked DNA," viral vectors which infect, for example, the skin or hair follicles when applied topically, or by other methods known in the art. Sections of the skin, preferably containing hair follicles, can then be made the basis for histoculture.

The invention also includes treating cells with a wide variety of beneficial or otherwise therapeutic compounds in addition to the tyrosinase expression system. The therapeutic compounds can be nucleic acids, hormones, proteins, enzymes, vitamins and other biochemical co-factors deemed to provide a therapeutic effect upon a hair or skin cell's growth, condition, color and the like. Particularly preferred are agents which improve the growth of the hair shaft, agents which stimulate the production of hair coloring pigments in the hair follicle, agents which replace pigment in the follicle cell or hair shaft (i.e., restore hair color), agents which stimulate hair growth, and agents which prevent hair loss. Most preferred are cell cycle inhibitors which prevent alopecia. Other agents useful in conditions of hair loss (alopecia) are those which stimulate hair growth, or those which inhibit the hair loss. Hair growth stimulators are generally well known, and include minoxidil, cyclosporin and the like known hair growth stimulators.

The invention additionally contemplates the administration of any gene beneficial to hair follicles through viral vector-mediated systems as described with respect to the tyrosinase locus herein. A gene is beneficial to hair follicles where it confers, upon selective delivery to the hair follicles by the present methods, a beneficial effect upon the hair follicle. Exemplary beneficial genes include genes normally and preferentially expressed in hair follicle, and therefore important for normal gene function. Beneficial genes can be identified by any of a variety of molecular biological methods. For example, a cDNA library of expressed genes can be prepared from hair follicle tissue supporting healthy hair, and can be enriched by subtractive hybridization against a cDNA library derived from a non-hair-producing or vellus-hair-producing follicle tissue, thereby producing a library of cDNA molecules whose expression is specific to hair follicles. Individual cDNA molecules from the hair specific cDNA library can be further screened for therapeutic effectiveness using the skin histoculture assay described in U.S. Pat. 5,641,508.

In one preferred embodiment, the invention contemplates bicistronic sequence for use in a method for restoring hair color in mammals, particularly human, in which the hair color is graying for any of a variety of reasons, including age. The method comprises applying a therapeutically effective amount of the bicistronic sequence of the invention to a skin area on the mammal having a plurality of hair follicles which exhibit fading or graying hair color. A "therapeutically effective" amount is the amount of a nucleic acid needed for the expression of tyrosinase and the production of melanin in the cells of the hair follicles. We also describe the delivery of cell cycle inhibitors such as p21 to hair follicles in order to prevent or inhibit alopecia. As is understood in the art, because cells in the hair matrix multiply rapidly -- i.e., are in anagen -- they are adversely affected by toxic agents that target rapidly dividing cells, such as those use in chemotherapy. By inhibiting the multiplication of these cells -- i.e., by supplying cell cycle inhibitors -- the effect of these agents on hair cells can be mitigated. We describe a method for genetic modification of hair cells by delivering the gene encoding a cell cycle inhibitor selectively to the hair follicle and effecting its expression. Thus, as described in the Examples below, the gene encoding the cell cycle inhibitor p21 (which has previously been cloned and sequenced) can be selectively delivered and expressed in these cells. Thus, the cells are placed in a state of telogen or a resting phase which effectively immunizes them from the effects of chemotherapeutic agents such as doxorubicin, cytoxain and the like.

Application of the bicistronic sequences or other expression system of the invention can be repeated at defined intervals to provide prolonged effectiveness, as needed.

As used herein, terms such as "pharmaceutically acceptable" and the like refer to compositions, carriers, diluents and reagents, and represents that the materials are capable of administration upon a mammal or human without the production of undesirable physiological effects such as nausea, dizziness, gastric upset and the like.

The preparation of a pharmacological composition that contains active ingredients dispersed therein is well understood in the art. Typically such compositions are prepared as sterile compositions either as liquid solutions or suspensions, aqueous or non-aqueous, however, suspensions in liquid prior to use can also be prepared.

The active ingredient can be mixed with excipients which are pharmaceutically acceptable and compatible with the active ingredient and in amounts suitable for use in the therapeutic methods described herein. Suitable excipients are, for example, water, saline, dextrose, glycerol, ethanol or the like and combinations thereof. In addition, if desired, the composition can contain minor amounts of auxiliary substances such as wetting or emulsifying agents, pH buffering agents and the like which enhance the effectiveness of the active ingredient. Liquid compositions can also contain liquid phases in addition to and to the exclusion of water. Exemplary of such additional liquid phases are glycerin, vegetable oils such as cottonseed oil, organic esters such as ethyl oleate, and water-oil emulsions.

A therapeutic composition contains a therapeutically effective amount of the invention expression system and, if present, other beneficial materials which are predetermined in amounts calculated to achieve the desired effects, i.e., to effectively affect the pigmentation of the skin or hair cells or inhibit alopecia. Thus, an effective amount can be measured by improvements in one or more symptoms associated skin or hair cells in the subject.

The dosage can be adjusted by the individual physician in the event of any complication. The compositions are administered in a manner compatible with the dosage formulation, and in a therapeutically effective amount. The quantity to be administered depends on the subject to be treated, capacity of the subject's system to utilize the active ingredient, and degree of therapeutic effect desired. Precise amounts of active ingredient required to be administered depend on the judgement of the practitioner and are peculiar to each individual. Suitable regimes for administration are also variable, but are typified by an initial administration followed by repeated doses at one or more hour intervals by a subsequent administration.

DNA segments (i.e., synthetic oligonucleotides) used to produce a larger DNA segment that comprises the bicistronic coding region of the invention can easily be synthesized by chemical techniques, for example, the phosphotriester method of Matteucci, *et al.* (J. Am. Chem. Soc., 103:3185-3191, 1981) or using automated synthesis methods. In addition, larger DNA segments can readily be prepared from smaller DNA segments by well known methods, such as synthesis of a group of oligonucleotides that define the DNA segment, followed by hybridization and ligation of oligonucleotides to build the complete segment. The required sequences can also be amplified from native coding regions, for example.

As used herein, the term "vector" refers to a DNA molecule capable of entering and modifying a cell. The choice of vector comprising a DNA segment of the present invention is dependent on the mode of administration. The vector may be simply naked DNA, a plasmid or a viral vector. The vector may be an "expression vector" -- i.e., it may contain control sequences for expression operatively linked, for example, to the bicistronic cassette of the invention.

Expression vectors compatible with mammalian cells, and particularly hair follicle cells, are well known in the art and are available from several commercial sources. Typically, such vectors contain convenient restriction sites for insertion of the desired DNA segment, and provide the signals required for gene expression in a mammalian cell. Typical of such vectors are the pREP series vectors and pEBVhis available from Invitrogen (San Diego, Calif.), the vectors pTDT1 (ATCC #31255), pCP1 (ATCC #37351) and pJ4W (ATCC #37720) available from the American Type Culture Collection (ATCC) and the like mammalian expression vectors.

When used in regard to correcting hair pigmentation disorders, particularly preferred are mammalian expression vectors which allow the expression of the gene in a tissue-specific manner, in this case by the action of a regulatory promoter that will limit gene expression to hair follicle cells.

Successfully modified hair follicle cells, i.e., follicle cells that contain the bicistronic cassette of the present invention, can be identified by well known techniques, for example, using a hybridization method such as that described by Southern, *J Mol Biol* (1975) 98:503 or Berent *et al. Biotech* (1985) 3:208. In addition to directly assaying for the presence of the nucleotide sequence, successful transformation can be confirmed by well known immunological methods for the presence of expressed protein.

Alternatively, successful transformation of the target tissue can be confirmed by evaluation of the target tissue for indicia of function exerted by the administered. For example, where the compound is a nucleic acid expressing tyrosinase, as described in the Examples, the exerted function of pigmentation, or the presence of tyrosinase activity or enzymatic conversion of L-dopa to product can be detected directly in the target tissue.

The following examples are intended to illustrate but not to limit the invention.

### Example 1

### Cloning of the Streptomyces tyrosinase gene, the upstream ORF-438 and the Internal Ribosome Entry Site (IRES)

The sequences encoding the *Streptomyces antibioticus* tyrosinase gene and ORF-438 were amplified by PCR from plasmid pIJ702 obtained from the American Type Culture Collection (ATCC #35287). Katz, E. *et al.* J. Gen. Microbiol. (1983) 129:2703-2714.

Oligomers for PCR amplification were designed according to the sequence of *S. antibioticus* tyrosinase gene and ORF-438 cDNA. Beman, V. *et al.* Gene, (1985) 37:101-110. In order to enhance expression of the bacterial gene in mammalian cells, the ORF-438 and tyrosinase-gene TGA termination codons were altered to TAA. The Kozak consensus sequence, GCCGCCACC, was added upstream, immediately preceding the ATG initiator codon in each case to facilitate translation efficiency.

The sequence of the ORF-438 upstream primer, which included the Kozak consensus sequence was 5'-CGGAATTCGCCGCCACCATGCCGGAACTCACCCGTC-3'. The downstream primer sequence was 5'-GGCTGATCATTAGTTGGAGGGAAGGGAGGAGC-3'. The sequence of the tyrosinase upstream primer, which includes the Kozak consensus sequence was

5'-CTCGAGGCCGCCGCCATGACCGTCCGCAAGAACCA-3'.

The downstream primer sequence was 5'-GGATCCTTAGACGTCGAAGGTGTAGTGC-3'. The PCR reaction conditions for both ORF-438 and tyrosinase were as follows: first denaturation at 97 °C for 10 min.; then 10 cycles of denaturation at 97 °C for 30 s; annealing at 66 °C for 30 s; and extension at 72 °C for 45 s; then a final extension at 72 °C for 10 min.

PCR oligomers were designed according to the sequence of the internal ribosome entry site (IRES) contained in the retroviral vector pLISN, obtained from Clonetech (Palo Alto, CA). The sequence of the upstream primer was 5'-GGCTGATCATTCGCCCCTCTCCCTCCCC-3'. The downstream primer sequence was

5'-AGCGGCCATTATCATCGTGTTTTTCAAAGG-3'.

The IRES gene was amplified by PCR from pLXIN as the template. The PCR reaction conditions were as follows: first denaturation at 96°C for 10 min; then 30 cycles of denaturation at 94 °C for 30 s; annealing at 50 °C for 30 s; and extension at 72 °C for 45 s; then a final extension at 72 °C for 10 min.

Electrophoretic analysis demonstrated that the amplified products had the predicted sizes of 438 bp, 800 bp and 580 bp for ORF-438, tyrosinase and IRES, respectively.

### Example 2

### Retroviral Vector pLmelSN Construction and Packaging

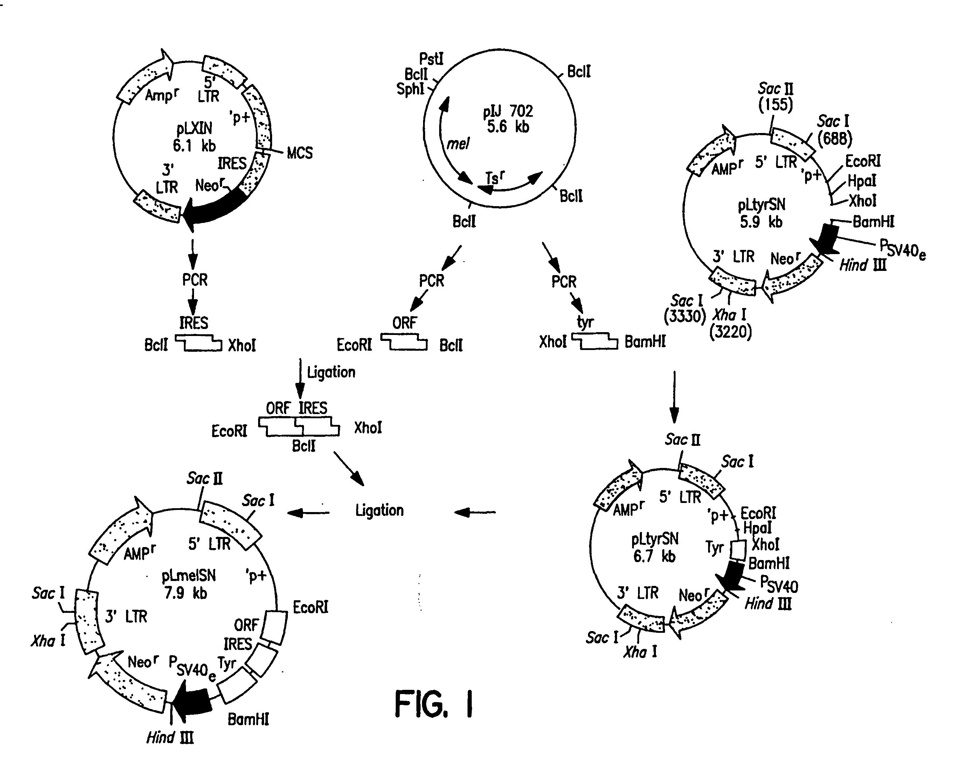

The construction of pL*mel*SN is shown in Figure 1. Retroviral vector pLXSN (Clonetech, Palo Alto, CA) is a murine leukemia virus-based vector containing two promoters: the 5'-long terminal repeat (5'-LTR) to control the inserted genes and the SV40 promoter to control neomycin phosphotransferase (neoR). The 800-bp tyrosinase PCR product was digested by XhoI and inserted into the HapI/XhoI cloning site of pLXSN to obtain pL*tyr*SN. The ORF-438 and IRES PCR products were ligated at the Bcl I site and then inserted into the EcoRI/XhoI cloning site of pL*tyr*SN to obtain pL*mel*SN. Both the ORF-438 and tyrosinase genes are driven by the Moloney murine leukemia virus 5'-LTR in pL*mel*SN. See Fig. 1. The bicistronic sequence containing the ORF-438, IRES and tyrosinase genes under control of the 5'-LTR promoter is shown in Figure 2.

pL*mel*SN was transfected into the PT67 packaging cell line using lipoTAXI (Clonetech, Palo Alto, CA; Stratagene, San Diego, CA). The transfected PT67 cell line was selected in DMEM medium containing 0.4 mg/ml G418 (Gibco BRL). The G418-resistant cells were cloned and expanded. After two weeks of selective culturing with G418, positive transfected cells, PT67-*mel*, were obtained.

### Example 3

### Expression in PT67-mel Cells

In order to confirm the expression of both the ORF-438 and the tyrosinase gene, RT-PCR analysis was used to detect their mRNA in the transfected packaging cells. The RT-PCR products demonstrated that the tyrosinase and ORF-438 genes from *S. antibioticus* were specifically amplified products from the total RNA of pL*mel*SN-transduced PT7-cells. As a control, mouse β-actin from both PT67-*mel* and PT67 cells was amplified by the RT-PCR. Electrophoretic analysis demonstrated that the amplified products from PT67-*mel* cells had the predicted sizes of 800 bp and 438 bp for tyrosinase and ORF-438, respectively. The RT-PCR reaction with total RNA from uninfected PT67 cells did not amplify these fragments.

In more detail, PT67-*mel* cells were digested with trypsin and pelleted by centrifugation. Total RNA was extracted by the guanidium thiocyanate method (MicroRNA Reagent Kit, Stratagene, San Diego, CA). RNA was quantified by measuring the absorbance at 260 nm. Approximately 10 µg of total RNA was reverse transcribed to first cDNA chains. Reverse transcription was carried out in 20 µl of first-strand buffer, 500 µM of each dNTP, and 20 units of AMV reverse transcriptase (Stratagene, San Diego, CA). The PCR primer for the first strand was pORF-438 antisense and pTyr antisense. Samples were incubated at 42 °C for 50 min. The products of the reverse transcription were amplified by the PCR reaction. Mouse β-actin was used as a standard to control the quality of the RNA (Stratagene, San Diego, CA).

### Example 4

### Tyrosinase Activity Assay

The transfected packaging cells were screened for the expression of active tyrosinase protein by measuring tyrosinase activity in the lysates of clones of G418-resistant packaging cells. Tyrosinase activity was assessed by the method described by Nakajima *et al. Pigment Cell Res* (1998) 11:12-17. The pL*mel*SN-infected PT67 packaging cells were plated at a density of 2,000 cells/well in 96 well plates. After 24 hours of culture, the packaging cells were washed with PBS and lysed with 1% Triton-100 (45 µl/well). After mixing the lysates by shaking, 5 µl of 10 mM L-DOPA were added to each well. Following incubation of the culture at 37 °C for 30 min., the absorbance was spectrophotometrically measured at 490 nm.

The DOPA-oxidase reaction was also used to detect melanin production in intact transfected PT67 cells. The cells were incubated with 1 mg/ml of DOPA and 2 mg/ml of tyrosine in PBS (pH 7.4) for 12 hr at 37 °C as previously described. See Kugelman, T. *et al.* J. Invest Dermatol (1961) 37:66-73.

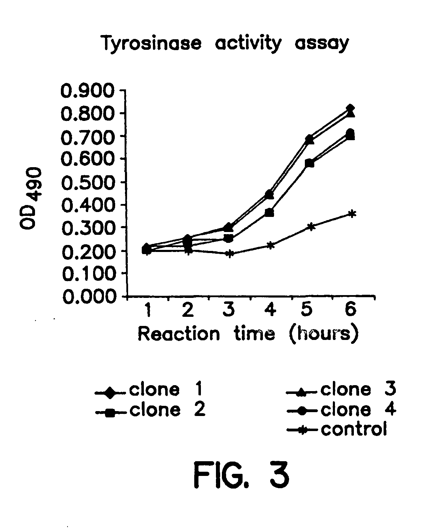

Using the same conditions, cell supernatants were measured for melanin content at 490 nm. Figure 3 shows PT67 clones 1-4 gentrate more melanin than control PT67. The tyrosinase-positive cells were identified by production of brown-colored melanin granules observed with brightfield microscopy. The brown pigment granules were observed only in pL*mel*SN-transfected cells.

### Example 5

### Retroviral Infection of Amelanotic Melanoma 695T Cells

Human amelanotic melanoma 695T cells (ATCC) were infected for 12 hours with retroviral supernatants containing 8 µg/ ml polybrene (Sigma, St. Louis, MO). Fresh MEM medium replaced the supernatant. The cultures were then incubated for 48 hours. Cells were then selected in MEM medium containing 0.4 mg/ml G418 for two weeks. Individual clones were isolated and expanded.

Brown melanin pigments were produced in the infected 695T cells. These brown melanin granules could be seen without the DOPA reaction. No pigment granules were seen in the untransduced 695T cells.

### Example 6

### Culture of Albino-Mouse Anagen Hair Follicles

Female albino mice C57BL/6J-Tyrosinase (c-2J), 8 week old, were purchased from the Jackson Laboratory. The growth phase of the hair cycle (anagen) was induced in the back skin, which had all follicles in the resting phase of the hair cycle (telogen). After general anesthesia, a warm wax/rosin mixture was applied and then peeled off the skin, depilating all telogen hair shafts and thereby inducing the follicles to enter anagen. See Schilli *J Invest Dermatol* (1998) 111:598-604.

On day-6 post-depilation, when all hair follicles were in anagen, back skins of the mice were collected after sacrifice. Small pieces of the mouse skin (2 x 5 x 2 mm) were cut with a scissors, and washed three times in HBSS. The harvested skin was incubated at 37 °C in MEM with antibiotics (100 µg gentamycin per ml, 10 µg ciprofroxacin per ml, 2.5 µg amphotericin-B per ml, 100 IU-100 µg penicillin-streptomycin per ml) for 30 min. (Sigma). The skins were washed three times with HBSS medium to remove residual antibiotics and put into collagen-containing gels for histoculture in Eagle's minimum essential medium (MEM) supplemented with 10% fetal bovine serum and gentamycin. Cultures were maintained at 37 °C in a gassed incubator with 5% CO₂.

### Example 7

### Infection of Cultured Albino-Mouse Hair Follicles

The histocultured albino-mouse skin of Example 6 was co-cultured with *PT67-mel* cells as follows:

The *PT67-mel* cells from the highest producing clone were counted, seeded in 24-well plates, and grown at 37 °C until 80% confluence, and co-cultured with the histocultured albino skin in 24-well plates for 12, 24 and 72 hours. The histocultured skins were then mono-cultured in 24-well plates with fresh MEM medium and incubated for an additional 4-6 days. Small pieces of virus-infected skin were sampled at random. Fresh and frozen sections were prepared by standard techniques.

Melanin was observed in the hair matrix deep in the hair bulbs of the histocultures four days after retroviral infection. Melanin was also found in the upper part of the hair follicles and could be observed in both the hair matrix and hair shaft six days after infection.

In the initial experiments which involved co-culturing histocultured albino mouse skin with PT67*-mel* cells for 24 hours, approximately 2.5-15% of the skin histocultures contained melanin-producing hair follicles. No melanin was observed in histocultured albino-mouse skin not co-cultured with PT67-*mel*.

A time-course experiment was then carried out to determine if longer incubation times of the co-cultures of the albino skin and PT67-*mel* increased the efficiency of tyrosinase infection. The efficiency of infection of the histocultured skin was significantly increased with time of co-culture. After 12 hours co-culture, 7% (2 of 30 pieces) of skin produced melanin, and 15% of hair follicles (6 of 40) produced melanin. After 24 hours co-culture, 25% of skin pieces (5 of 20) produced melanin, as did 35% (28 of 80) of hair follicles. After 72 hours co-culture, 60% of skin pieces (12 of 20) produced melanin as did 53% (42 of 80) of hair follicles.

These results suggest that the virus titer and time exposure to virus can affect the transduction frequency.

### Example 8

### Cloning of Human p21 Gene

The nucleotide sequence encoding the human p21 gene was amplified by PCR from plasmid MHP-p21, Zhang *et al. Gene* (1994) 3:1750-1758 (1994). Oligomers were designed according to the sequence of the human p21 gene. Xiong *et al.* Nature (1993) 366:701. The upstream primer was 5'-CCG CTC GAG ATG TCA GAA CCG GCT GG-3'. The downstream primer was 5'-CGC GGA TTC TTA GGG CTT CCT CTT GGA CT-3'. The PCR primer reaction conditions were as follows: first denaturation at 94 °C for 10 min; then 30 cycles of denaturation at 94 °C for 30 s; annealing at 50 °C for 30 s; and extension at 72 °C for 45 s; then a final extension at 72° for 10 min.

Electrophoretic analysis demonstrated that the amplified products had the predicted size of 500 bp.

### Example 9

### Construction of pEGFP-p21

The vector pEGFP-C₃ (Clonetech, Palo Alto, CA), encodes a red-shifted variant of wild-type GFP that has been optimized for brighter fluorescence and higher expression in mammalian cells. The multiple cloning site (MCS) in pEGFP-C₃ is between the EGFP coding sequences and the SV40 polyA. Genes cloned into the MCS will be expressed as fusion to the C-terminus of EGFP if they are in the same reading frame (Neo^{R}) contained in the vector allows stable transfected eukaryotic cells to be selected using G418.

The 500 bp p21 amplified gene was cloned into the XhoI/BamHI cloning site of the pEGFP-C₃ vector to obtain pEGFP-p21, and correct insertion confirmed by restriction enzyme analysis.

### Example 10

### Histoculture of Skin and Transfection of Cultured Skin with pEGFP-p21 Vector

Baby balb-c mice (2 weeks) were treated with hair remover. Small pieces of mouse skin (2 x 5 x 2 mm) were cut with a scissors and put onto collagen-containing gels in histoculture in Eagle's minimum essential medium (MEM) supplemented with 10% fetal bovine serum and gentamycin, as described by Li *et al.* PNAS USA (1991) 88:1908-1912. Cultures were maintained at 37 °C in a gassed incubator with 5% CO₂. Liposome interaction with the skin was initiated after 24 hours of histoculture.

40 ml of LipoTAXI transfection reagent (Stratagene, San Diego, CA) were added to 20ml (10 µg) of pEGFP-p21 plasmid DNA, then mixed and incubated for 30 min. at room temperature. 400 µl of serum-free MEM was added to the mixture, then transferred to the skin-culture dish with swirling and the mixtures were incubated for 4 hours at 37 °C. The medium was replaced with 2 ml of MEM with 10% serum and incubated for 48 hours at 37° in 5% CO₂.

A Nikon fluorescence microscope, equipped with aGFP cubes was used to observe expression. The EGFP-p21 gene was expressed selectively in hair follicles as visualized by bright GFP fluorescence.

## Claims

1. A nucleic acid molecule for efficient production of a functional tyrosinase in mammalian cells, which molecule comprises a bicistronic nucleotide sequence comprising a *Streptomyces* tyrosinase-encoding nucleotide sequence and a nucleotide sequence encoding a Streptomyces ORF-438 coupled through an internal ribosome entry sequence (IRES).

2. The nucleic acid molecule of claim 1 wherein each of said coding sequences is operably linked to a Kozak consensus sequence.

3. The nucleic acid molecule of claim 1 wherein said coding sequences have been modified to contain TAA termination codons.

4. The nucleic acid molecule of any one of claims 1 to 3 wherein said coding sequences are operably linked to one or more control sequences that direct expression of said coding sequences.

5. The nucleic acid molecule of claim 4 which is a vector.

6. The nucleic acid molecule of claim 5 which is a viral vector.

7. The nucleic acid molecule of claim 6 which is a retroviral vector.

8. Isolated mammalian cells modified to contain the nucleic acid molecule of any one of claims 1 to 7.

9. The cells of claim 8 which are hair follicle cells.

10. The cells of claim 9 which are contained in a skin histoculture.

11. The cells of claim 10 which have been synchronized in anagen.

12. A model system for evaluating the effect of compounds or protocols on pigmentation, which comprises the histoculture of claim 10 or 11.

13. The nucleic acid molecule of any one of claims 1 to 7 for use in a method of treatment of the human or animal body by therapy.

14. Use of the nucleic acid molecule of any of claims 1 to 7 in the manufacture of a medicament for the treatment of a disorder of tyrosinase gene expression and/or melanin biosynthesis.

15. The use of claim 14 wherein said treatment comprises treating cells exhibiting the disorder by direct transfection, an electric pulse, lipofection or viral infection.

16. The use of claim 15 wherein the cells are contained in a mammalian subject.

17. The use of any one of claims 14 to 16 wherein said medicament is suitable for topical administration.

18. The use of claim 17 wherein said topical administration is by use of naked DNA, a viral vector or a liposomal composition.

19. The use of any one of claims 15 to 18 wherein the cells are hair follicle cells or skin cells.

20. A method of determining the effect of a compound on pigmentation comprising:
(i) culturing the system of claim 12 in the presence and absence of said compound; and
(ii) observing an increase or decrease in melanin production, if any, in the presence compared to the absence of the compound.

## Patentansprüche

1. Ein Nukleinsäuremolekül zum effizienten Erzeugen einer funktionellen Tyrosinase in Säugerzellen, wobei das Molekül eine bicistronische Nukleotidsequenz umfasst, die eine Streptomyces-Tyrosinase-kodierende Nukleotidsequenz und eine Nukleotidsequenz, die ein Streptomyces-ORF-438 kodiert, umfasst, die durch eine interne Ribosomen-Eintrittsstelle-Sequenz (IRES) gekoppelt sind.

2. Nukleinsäuremolekül nach Anspruch 1, bei dem jede der kodierenden Sequenzen funktionell mit einer Kozak-Consensus-Sequenz verbunden ist.

3. Nukleinsäuremolekül nach Anspruch 1, bei dem die kodierenden Sequenzen so modifiziert worden sind, dass sie TAA-Stoppcodons enthalten.

4. Nukleinsäuremolekül nach einem der Ansprüche 1 bis 3, bei dem die kodierenden Sequenzen funktionell mit einer oder mehreren Steuersequenz(en) verbunden sind, welche die Expression der kodierenden Sequenzen steuern.

5. Nukleinsäuremolekül nach Anspruch 4, bei dem es sich um einen Vektor handelt.

6. Nukleinsäuremolekül nach Anspruch 5, bei dem es sich um einen viralen Vektor handelt.

7. Nukleinsäuremolekül nach Anspruch 6, bei dem es sich um einen retroviralen Vektor handelt.

8. Isolierte Säugerzellen, die so modifiziert sind, dass sie das Nukleinsäuremolekül nach einem der Ansprüche 1 bis 7 enthalten.

9. Zellen nach Anspruch 8, bei denen es sich um Haarfollikelzellen handelt.

10. Zellen nach Anspruch 9, die in einer Haut-Gewebekultur enthalten sind.

11. Zellen nach Anspruch 10, die im Anagen synchronisiert worden sind.

12. Ein Modellsystem zur Bewertung des Effekts von Verbindungen oder Protokollen auf die Pigmentierung, das die Gewebekultur nach Anspruch 10 oder 11 umfasst.

13. Nukleinsäuremolekül nach einem der Ansprüche 1 bis 7 zur Verwendung in einem Verfahren zur Behandlung des menschlichen oder tierischen Körpers durch eine Therapie.

14. Verwendung des Nukleinsäuremoleküls nach einem der Ansprüche 1 bis 7 bei der Herstellung eines Medikaments zur Behandlung einer Störung der Tyrosinasegenexpression und/oder der Melaninbiosynthese.

15. Verwendung nach Anspruch 14, bei der die Behandlung eine Behandlung von Zellen, welche die Störung aufweisen, durch direkte Transfektion, einen elektrischen Impuls, eine Lipofektion oder eine virale Infektion umfasst.

16. Verwendung nach Anspruch 15, bei der die Zellen in einem Säuger enthalten sind.

17. Verwendung nach einem der Ansprüche 14 bis 16, bei der das Medikament für eine topische Verabreichung geeignet ist.

18. Verwendung nach Anspruch 17, bei der die topische Verabreichung durch die Verwendung nackter DNA, eines viralen Vektors oder einer liposomalen Zusammensetzung stattfindet.

19. Verwendung nach einem der Ansprüche 15 bis 18, bei der die Zellen Haarfollikelzellen oder Hautzellen sind.

20. Ein Verfahren zur Bestimmung des Effekts einer Verbindung auf die Pigmentierung, umfassend:
(i) Kultivieren des Systems nach Anspruch 12 in der Gegenwart der Verbindung und ohne die Verbindung und
(ii) Untersuchen einer Zunahme oder Abnahme der Melaninerzeugung, falls eine solche vorliegt, in der Gegenwart der Verbindung im Vergleich zum Fehlen der Verbindung.

## Revendications

1. Molécule d'acide nucléique pour la production efficace d'une tyrosinase fonctionnelle dans des cellules de mammifères, laquelle molécule comprend une séquence nucléotidique bicistronique comprenant une séquence nucléotidique codant une tyrosinase de *Streptomyces* et une séquence nucléotidique codant un ORF-438 de *Streptomyces* couplé par l'intermédiaire d'un site interne d'entrée du ribosome (IRES).

2. Molécule d'acide nucléique de la revendication 1, dans laquelle chacune desdites séquences codantes est liée de façon opérationnelle à une séquence consensus de Kozak.

3. Molécule d'acide nucléique de la revendication 1, dans laquelle lesdites séquences codantes ont été modifiées pour contenir des codons de terminaison TAA.

4. Molécule d'acide nucléique de l'une quelconque des revendications 1 à 3, dans laquelle lesdites séquences codantes sont liées de façon opérationnelle à une ou plusieurs séquences de contrôle qui dirigent l'expression desdites séquences codantes.

5. Molécule d'acide nucléique de la revendication 4 qui est un vecteur.

6. Molécule d'acide nucléique de la revendication 5 qui est un vecteur viral.

7. Molécule d'acide nucléique de la revendication 6, qui est un vecteur rétroviral.

8. Cellules de mammifère isolées modifiées pour contenir la molécule d'acide nucléique de l'une quelconque des revendications 1 à 7.

9. Cellules de la revendication 8, qui sont des cellules de follicules pileux.

10. Cellules de la revendication 9, qui sont contenues dans une histoculture de peau.

11. Cellules de la revendication 10, qui ont été synchronisées en anagène.

12. Système modèle pour évaluer l'effet de composés ou de protocoles sur la pigmentation, qui comprend l'histoculture de la revendication 10 ou 11.

13. Molécule d'acide nucléique de l'une quelconque des revendications 1 à 7, destinée à être utilisée dans un procédé de traitement de l'organisme humain ou animal par thérapie.

14. Utilisation de la molécule d'acide nucléique de l'une quelconque des revendications 1 à 7, dans la fabrication d'un médicament pour le traitement d'un trouble de l'expression du gène tyrosinase et/ou de la biosynthèse de mélanine.

15. Utilisation de la revendication 14, dans laquelle ledit traitement comprend le traitement de cellules présentant le trouble par transfection directe, une impulsion électrique, lipofection ou infection virale.

16. Utilisation de la revendication 15, dans laquelle les cellules sont contenues dans un sujet mammifère.

17. Utilisation de l'une quelconque des revendications 14 à 16, dans laquelle ledit médicament convient à une administration topique.

18. Utilisation de la revendication 17, dans laquelle ladite administration topique se fait par l'utilisation d'ADN nu, d'un vecteur viral ou d'une composition liposomique.

19. Utilisation de l'une quelconque des revendications 15 à 18, dans laquelle les cellules sont des cellules de follicules pileux ou des cellules cutanées.

20. Procédé de détermination de l'effet d'un composé sur la pigmentation comprenant : et
(i) la culture du système de la revendication 12 en présence et en l'absence dudit composé ;
(ii) l'observation d'une augmentation ou d'une diminution de la production de mélanine, le cas échéant, en présence du composé par rapport à la production en l'absence du composé.
